(19) Europäisches Patentamt
European Patent Office
Office européen des brevets

(11) Veröffentlichungsnummer: **0 158 075**
**A1**

# (12) EUROPÄISCHE PATENTANMELDUNG

(21) Anmeldenummer: 85102045.3

(22) Anmeldetag: 25.02.85

(51) Int. Cl.⁴: A 61 K 31/53
C 07 D 251/34, C 07 D 251/38
C 07 D 417/12

(30) Priorität: 09.03.84 DE 3408768

(43) Veröffentlichungstag der Anmeldung: 16.10.85 Patentblatt 85/42

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Brunner, Helmut, Prof. Dr.
Waldstrasse 10
D-4018 Langenfeld(DE)

(72) Erfinder: Haberkorn, Axel, Dr.
Fuhlrottstrasse 99
D-5600 Wuppertal(DE)

(72) Erfinder: Böshagen, Horst, Dr.
Wiesenstrasse 4
D-5657 Haan(DE)

(72) Erfinder: Stoltefuss, Jürgen, Dipl.-Ing.
Parkstrasse 20
D-5657 Haan(DE)

(72) Erfinder: Kume, Toyohiko
6-7-8, Asahigaoka
Hino-shi Tokyo(JP)

(54) **Immunstimulierende Mittel.**

(57) Die vorliegende Erfindung betrifft immunstimulierende Mittel, ihre Verwendung und Anwendung, die Verbindungen der Formel I enthalten:

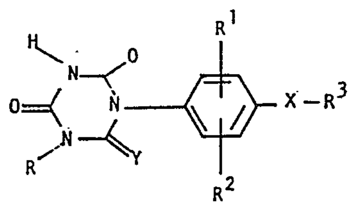

(I)

in welcher

R, $R^1$, $R^2$, $R^3$, X, Y die in der Beschreibung angegebene Bedeutung haben. Die Erfindung betrifft ferner ein Verfahren zur Intervallbehandlung zur Bekämpfung von Coccidien-Erkrankungen.

EP 0 158 075 A1

Croydon Printing Company Ltd.

BAYER AKTIENGESELLSCHAFT 5090 Leverkusen, Bayerwerk
Konzernverwaltung RP
Patentabteilung Rt/ABc

Immunstimulierende Mittel

Die vorliegende Erfindung betrifft immunstimulierende Mittel, ihre Verwendung sowie Methoden ihrer Anwendung.

Es ist bekannt, Impfstoffe mit Adjuvantien, d.h. die Antikörperbildung verstärkenden Substanzen, zu verabreichen. So wird für diesen Zweck Freunds'sches komplettes Adjuvans eingesetzt. Es handelt sich dabei um eine Wasser-in-Öl-Emulsion, der abgetötete Mykobakterien beigefügt sind. Ferner können abgetötete Mikrobakterien die zell-vermittelte Immunität und die Makrophagentätigkeit stimulieren.

Es ist ferner bekannt, daß N-Acetyl-muramyl-L-alanyl-D-glutamin-n-butylester, ein synthetisch hergestelltes Adjuvans ist, das die unspezifische Infektionsabwehr stimuliert (Robert Koch Stiftung e.V. Beiträge und Mitteilungen Vol. 5/1983, S. 31-38).

Es wurde gefunden, daß Verbindungen der Formel I

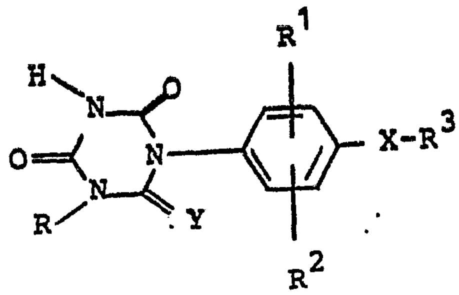

(I)

in welcher

R für Alkyl steht,

$R^1$ für Wasserstoff, Alkyl, Halogen, Halogenalkyl, Alkoxy steht,

$R^2$ für Wasserstoff, Alkyl, Halogen, Halogenalkyl, Alkoxy steht,

$R^3$ für gegebenenfalls substituiertes Aryl oder Heteroaryl steht,

X für O oder S steht,

Y für O oder S steht,

immunstimulierende Wirkungen besitzen.

Die erfindungsgemäß verwendbaren Verbindungen steigern die Antikörpersynthese des Immunsystems und verstärken die unspezifische körpereigene Abwehr. Sie lassen sich sowohl in der Human- wie in der Veterinärmedizin einsetzen. Es war überraschend, daß diese Substanzen ausgezeichnete immunstimulierende Wirkungen besitzen. Sie führen zu einer Verlängerung der Überlebenszeit und Erhöhung der Überlebensrate von Mäusen nach letaler bakterieller Infektion.

0158075

Die Verbindungen der Formel I sind bekannt oder können nach an sich bekannten Methoden hergestellt werden (DE-OS 2 413 722; US 4 219 552).

Bevorzugt werden Verbindungen der Formel I eingesetzt, in welcher

R für $C_{1-4}$-Alkyl steht,

$R^1$ und $R^2$ gleich oder verschieden für Wasserstoff, $C_{1-4}$-Alkyl, Halogen wie insbesondere Fluor, Chlor oder Brom, $C_{1-4}$-Alkoxy und $C_{1-2}$-Halogenalkyl stehen,

$R^3$ für Phenyl steht, das gegebenenfalls substituiert ist durch Halogen insbesondere Fluor, Chlor, Brom, $No_2$, $C_{1-4}$-Alkyl, $C_{1-2}$-Halogenalkyl, $C_{1-4}$-Alkylmercapto, das gegebenenfalls durch Halogen substituiert ist, $C_{1-4}$-Alkoxy, das gegebenenfalls durch Halogen substituiert ist, $C_{1-4}$-Alkylsulfinyl, das gegebenenfalls durch Halogen substituiert ist, $C_{1-4}$-Alkylsulfonyl, das gegebenenfalls durch Halogen substituiert ist oder für einen Benzoxazolyl- oder Benzthiazolyl-Rest steht, der gegebenenfalls weitere Substituenten tragen kann wobei insbesondere die oben genannten in Frage kommen.

Besonders bevorzugt sind Verbindungen der Formel I, in welcher

R für Methyl steht,

$R^1$ und $R^2$ gleich oder verschieden sind und für Wasserstoff, Fluor, Chlor, Brom, Methyl, Trifluormethyl, Methoxy, Ethoxy stehen,

$R^3$ für Phenyl steht, das insbesondere in 4-Stellung durch Trifluormethylmercapto, Trifluormethylsulfinyl, Trifluormethylsulfonyl, Trifluormethoxy substituiert ist, ferner für eine Benzoxazolyl- oder Benzthiazolyl-Gruppe steht, die gegebenenfalls durch die Reste $R^1$ bzw. $R^2$ substituiert ist,

X und Y stehen bevorzugt für O.

Folgende Verbindungen seien beispielhaft genannt, ohne die Erfindung in irgendeiner Weise einzuschränken:

1-/3,5-Dichlor-4-(4'-trifluormethoxy-phenoxy)-phenyl/-3-methyl-1,3,5-triazin-2,4,6(1H, 3H, 5H)-trion,
1-/3,5-Dichlor-4-(4'-trifluormethylthio-phenoxy)-phenyl/-3-methyl-1,3,5-triazin-2,4,6(1H, 3H, 5H)-trion,
1-/4-(4'-Trifluormethylthio-phenoxy)-phenyl/-3-methyl-1,3,5-triazin-2,4,6(1H, 3H, 5H)-trion,

1-[3-Chlor-5-brom-4-(4'-trifluormethylthio-phenoxy)-phenyl]-3-methyl-1,3,5-triazin-2,4,6(1H, 3H, 5H)-trion,

1-[3,5-Dibrom-4-(4'-trifluormethylthio-phenoxy)-phenyl]-3-methyl-1,3,5-triazin-2,4,6(1H, 3H, 5H)-trion,

1-[3,5-Dichlor-4-(3'-methyl-4'-trifluormethylthio-phenoxy)-phenyl]-3-methyl-1,3,5-triazin-2,4,6(1H, 3H, 5H)-trion,

1-[4-(4'-Trifluormethylsulfonyl-phenoxy)-phenyl]-3-methyl-1,3,5-triazin-2,4,6(1H, 3H, 5H)-trion,

1-[3,5-Dichlor-4-(4'-trifluormethylsulfonyl-phenoxy)-phenyl]-3-methyl-1,3,5-triazin-2,4,6(1H, 3H, 5H)-trion,

1-[3-Chlor-5-brom-4-(4'-trifluormethylsulfonyl-phenoxy)-phenyl]-3-methyl-1,3,5-triazin-2,4,6(1H, 3H, 5H)-trion,

1-[3,5-Dichlor-4-(2'-Chlor-4'-trifluormethylsulfonyl-phenoxy)-phenyl]-3-methyl-1,3,5-triazin-2,4,6(1H, 3H, 5H)-trion,

1-[3-Methoxy-4-(4'-trifluormethylthio-phenoxy)-phenyl]-3-methyl-1,3,5-triazin-2,4,6,-(1H,3H,5H)-trion,

1-[4-(4'-Trifluormethylsulfinyl-phenoxy)-phenyl]-3-methyl-1,3,5-triazin-2,4,6(1H, 3H, 5H)-trion,

1-[3,5-Dichlor-4-(4'-trifluormethylsulfinyl-phenoxy)-phenyl]-3-methyl-1,3,5-triazin-2,4,6(1H, 3H, 5H)-trion,

1-[4-(3'-Trifluormethylsulfonyl-phenoxy)-3,5-dimethylphenyl]-3-methyl-1,3,5-triazin-2,4,6(1H, 3H, 5H)-trion,

1-[3-Chlor-4-(4'-trifluormethylsulfonyl-phenoxy)-phenyl]-3-methyl-1,3,5-triazin-2,4,6(1H, 3H, 5H)-trion,

1-[3-Methyl-4-(4'-trifluormethylthio-phenoxy)-phenyl]-3-methyl-1,3,5-triazin-2,4,6-(1H, 3H, 5H)-trion,

1-[3,5-Dichlor-4-(2'-Methyl-4'-trifluormethylsulfonyl-phenoxy)-phenyl]-3-methyl-1,3,5-triazin-2,4,6(1H, 3H, 5H)-trion,

1-[3-Chlor-5-methyl-4-(2'-Chlor-4'-trifluormethylsulfonyl-phenoxy)-phenyl]-3-methyl-1,3,5-triazin-2,4,6(1H, 3H, 5H)-trion,

1-[4-(4'-Trifluormethylsulfonyl-phenoxy)-3,5-dimethylphenyl]-3-methyl-1,3,5-triazin-2,4,6(1H, 3H, 5H)-trion,
1-[3-Chlor-5-methyl-4-(4'-trifluormethylsulfonyl-phenoxy)-phenyl]-3-methyl-1,3,5-triazin-2,4,6(1H, 3H, 5H)-trion,
1-[3-Chlor-4(2'-Chlor-4'-trifluormethylthio-phenoxy)-phenyl]-3-methyl-1,3,5-triazin-2,4,6(1H, 3H, 5H)-trion,
1-[3-Brom-4-(4'-trifluormethylsulfonyl-phenoxy)-phenyl]-3-methyl-1,3,5-triazin-2,4,6(1H, 3H, 5H)-trion,
1-[3-Chlor-5-trifluormethyl-4-(4'-trifluormethylsulfonyl-phenoxy)-phenyl]-3-methyl-1,3,5-triazin-2,4,6(1H, 3H, 5H)-trion,
1-[3,5-Dichlor-4-(4'-trifluormethylsulfonyl-phenoxy)-phenyl]-3-methyl-2-thioxo-4,6-dioxo-1,3,5(1H, 3H, 5H)-triazin,
1-[3-Methyl-4-(4'-trifluormethylthio-phenoxy)-phenyl]-3-methyl-2-thioxo-4,6-dioxo-1,3,5-(1H,3H,5H)-triazin.
1-[3-Methyl-4-(6-trifluormethyl-benzthiazol-2-yloxy)-phenyl]-3-methyl-1,3,5-triazin-2,4,6-(1H,3H,5H)-trion.

Besonders vorteilhaft werden die Wirkstoffe in einer Intervallbehandlung eingesetzt, d.h. die behandelten Lebewesen werden nur in den für den Erreger besonders empfänglichen Lebensphasen behandelt. Es ist dabei ausreichend, wenn die Behandlung in dieser Zeit an ein bis drei Tagen pro Woche durchgeführt wird.

Das heißt z.B. für Geflügel, daß eine Behandlung an 1-3, bevorzugt 1-2 Tagen jeweils in der 2., 3. und 4. Lebenswoche erfolgt.

Bei Säugetieren wird die Behandlung jeweils nach dem Absetzen vom Muttertier einmal pro Woche durchgeführt.

So wird bei Kaninchen, Schweinen oder Kälbern ab der 4.-6. Woche jeweils 1 x pro Woche behandelt.

Diese Intervallbehandlung kann eingesetzt werden zur Bekämpfung von Coccidienarten des Geflügels wie z.B. Eimeria tenella (Blinddarmcoccidiose des Huhn), E. acervulina, E. brunetti, E. maxima, E. mitis, E. necatrix und E. praecox (Dünndarmcoccidiose / Huhn). Sie ist ferner einsetzbar zur Prophylaxe und Behandlung von Coccidiose-Infektionen anderer Hausgeflügelarten sowie von Coccidieninfektionen von Säugetieren, wie z.B. des Kaninchen (E. stiedae / Lebercoccidiose, E. flavescens, E. magna, E. media, E. irresidua, E. perforans / Darmcoccidiose), der Schafe, Rinder und andere Haustiere, einschließlich Hund und Katze sowie von Labortieren wie der weißen Maus (E. falciformis) und der Ratte.

Die Wirkstoffe können in bekannter Weise in die üblichen Formulierungen übergeführt werden wie Praemixe zur Verabreichung mit dem Futter, Tabletten, Dragees, Kapseln, Suspensionen und Sirupe.

Die Verabreichung der Verbindungen erfolgt zwar gewöhnlich am zweckmäßigsten in oder mit dem Futter oder im Trinkwasser, die Verbindungen können aber auch einzelnen Tieren in Form von Tabletten, Arzneitränken, Kapseln oder dergleichen oder durch Injektion verabreicht werden. Die letztgenannten Verabreichungsmethoden sind nur gut zur Verabreichung an eine kleine Zahl von Tieren oder an Einzeltiere geeignet.

Ein wirkstoffhaltiges Futter wird mit den erfindungsgemäßen Verbindungen gewöhnlich in der Weise zubereitet, daß etwa 5 - 5000, vorzugsweise etwa 5 - 250 ppm Wirkstoff mit einem nährstoffmäßig ausgeglichenen Tierfutter, z.B. mit dem in dem folgenden Beispiel beschriebenen Kükenfutter, gründlich vermischt werden.

Wenn ein Konzentrat oder eine Vormischung zubereitet werden soll, die schließlich im Futter auf die obengenannten Werte verdünnt werden soll, werden im allgemeinen etwa 1 bis 30 %, vorzugsweise etwa 10 bis 20 Gewichtsprozent Wirkstoff mit einem eßbaren organischen oder anorganischen Träger, z.B. Maismehl oder Mais- und Sojabohnenmehl oder Mineralsalzen, die eine kleine Menge eines eßbaren Entstäubungsöls, z.B. Maisöl oder Sojabohnenöl enthalten, vermischt. Die so erhaltene Vormischung kann dann dem vollständigen Geflügelfutter vor der Verabreichung zugegeben werden.

Als Beispiel für die Verwendung der Wirkstoffe im Geflügelfutter kommt die folgende Zusammensetzung in Frage:

52,0000 % Futtergetreideschrot
17,9995 % Sojaschrot
5,0000 % Maiskleberfutter
5,0000 % Weizenvollmehl
3,0000 % Fischmehl
3,0000 % Tapiokamehl
3,0000 % Luzernegrasgrünmehl

2,0000 % Weizenkeime, zerkleinert
2,0000 % Sojaöl
1,6000 % Fischknochenmehl
1,5000 % Molkenpulver
1,4000 % kohlensaurer Futterkalk
1,0000 % phosphorsaurer Futterkalk
1,0000 % Melasse
0,5000 % Bierhefe
0,0015 % 1-[3,5-Dichlor-4-(4'-trifluormethylsulfonyl-phenoxy)-phenyl]-3-methyl-1,3,5-triazin-2,4,6(1H, 3H, 5H)-trion

---

100,0000 %

Für eine Anwendung der Wirkstoffe im Trinkwasser kommen wassermischbare Lösungen der Wirkstoffe, die ein oder mehrere polare Lösungsmittel enthalten und alkalisch reagieren, in Frage.

Zur Herstellung solcher Lösungen wird der Wirkstoff in einem polaren, wasserslöslichen Lösungsmittel gelöst, welches entweder alkalisch reagiert oder dem eine alkalische wasserlösliche Substanz zugefügt wird. Letztere wird vorteilhafter ebenfalls im Lösungsmittel gelöst, kann aber auch in dem Lösungmittel suspendiert sein und sich erst im Trinkwasser lösen. Dabei soll das Trinkwasser nach Zusatz der Wirkstofflösung einen pH-Wert von mehr als 7, vorzugsweise aber einen pH-Wert größer als pH 8 haben.

Die Lösung des Wirkstoff-Konzentrats sollte einen pH von 11 nicht überschreiten und einen pH von 8 nicht unterschreiten.

Die Konzentration des Wirkstoffes kann im Bereich von 0,5 - 50 % liegen, vorzugsweise aber in einem Bereich von 1-25 %.

Als Lösungsmittel kommen alle wasserlöslichen Lösungsmittel in Betracht, in denen der Wirkstof in genügender Konzentration löslich ist und die physiologisch unbedenklich sind.

Dies sind aus der Reihe der Alkohole ein - und mehrwertige wie z.B. Ethylalkohol, Isopropylalkohol, Benzylalkohol, Glycerin, Propylenglykol, Polyethylenglykole, Poly(oxoethylen)-poly(oxypropylen)-Polymere, basische Alkohole wie z.B. Mono-, Di- und Triethanolamin.

Außerdem sind geeignet Ketone z.B. Aceton oder Methylethylketon und aus der Reihe der Ester z.B. Milchsäureethylester. Andere Lösungsmittel wie N-Methylpyrrolidon, Dimethylacetamid, Dimethylformamid können ebenfalls eingesetzt werden.

Als Basen zur Einstellung des alkalischen pH-Wertes sind vorzugsweise organische Basen einzusetzen, z.B. basische Aminosäuren wie L- bzw. D,L-Arginin, L- bzw. D, L-Lysin, Methylglucosamin, Glucosamin, 2-Amino-2-hydroxymethyl-

propandiol-(1,3), Cholin, Piperazin. Auch Diamine sind hier geeignet z.B. bildet N, N, N', N'-tetrakis-(2-hydroxypropyl)-ethylen- diamin oder Polyether-Tetrol auf der Basis Ethylendiamin (M.G. 480-420, OH-Index 432-467) ebenfalls klare Lösungen im angegebenen pH-Bereich aus. Auch anorganische Basen, können eingesetzt werden, z.B. Ammoniak oder Natriumcarbonat - gegebenenfalls unter Zugabe von Wasser.

Substanzen, die sonst als Emulgatoren oder Solubilisatoren verwendet werden und in Wasser kolloidal löslich sind, können in diesem Falle wie polare Lösungsmittel eingesetzt werden, sofern ihnen noch ein basischer Hilfsstoff zugemischt wird.

Zur Herstellung der erfindungsgemäßen Lösungen werden die Substanzen in einen Behälter mit Rührwerk eingewogen und dann unter Erwärmen solange gerührt, bis eine klare Lösung entstanden ist.

Wassermischbare Lösungen der Wirkstoffe zur Anwendung im Trinkwasser sind z.B.:

Beispiel 1

2,5 g Wirkstoff werden zu 100 ml in Triethanolamin unter Erwärmen gelöst.

Die klare Lösung hat einen pH-Wert von 10,2.

Beispiel 2

2,5 g Wirkstoff
und 12,5 g Milchsäure werden zu 100 ml in Triethanolamin unter Erwärmen und Rühren gelöst.

Der pH der Lösung beträgt 8,3.

Beispiel 3

10,0 g Wirkstoff wird zu 100 ml in Monoethanolamin gelöst.

Die klare Lösung hat einen pH-Wert von 11.

Beispiel 4

| | | |
|---|---|---|
| Wirkstoff | | 5,0 g |
| Propylenglykol | | 50,0 g |
| Natriumcarbonat | | 5,0 g |
| Wasser | ad | 100 ml |

pH der Lösung 9,9.

Beispiel 5

5,0 g Wirkstoff
25,0 g D,L-Lysin Base
ad 100 ml Polyethylenglykol 400

pH der Lösung 9,8

Beispiel 6

25,0 g Wirkstoff
10,0 g Monoethanolamin
ad 100 ml N-Methylpyrrolidon

pH der Lösung 10,8.

Das Chemotherapeutikum kann für die Einzelbehandlung entweder als solches oder aber in Kombination mit pharmazeutisch annehmbaren Trägern zur Anwendung gelangen. Als Darreichungsform in Kombination mit verschiedenen inerten Trägern kommen Tabletten, Kapseln, Dragees, wäßrige Suspensionen, injizierbare Lösungen Elixiere, Sirupe und dergleichen in Betracht. Derartige Träger umfassen feste Verdünnungsmittel oder Füllstoffe, ein steriles wäßriges Medium sowie verschiedene nichttoxische organische Lösungsmittel und dergleichen. Selbstverständlich können die für eine orale Verabreichung in Betracht kommenden Tabletten und dergleichem mit Süßstoffzusatz und ähnlichem versehen werden. Die therapeutisch wirksame Verbindung soll im vorgenannten Fall in einer Konzentration von etwa 0,5 - 90 Gew.-% der Gesamtmischung vorhanden sein, d.h. in Mengen, die ausreichend sind, um den obengenannten Dosierungsspielraum zu erreichen.

Im Falle der oralen Anwendung können Tabletten selbstverständlich auch Zusätze wie Natriumcitrat, Calciumcarbonat und Dicalciumphosphat zusammen mit verschiedenen Zuschlagstoffen wie Stärke, vorzugsweise Kartoffelstärke und dergleichen und Bindemitteln wie Polyvinylpyrrolidon, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, kann der Wirkstoff mit verschiedenen Geschmacksaufbessern, Farbstoffen,

Emulgier- und/oder zusammen mit Verdünnungsmitteln wie Wasser, Ethanol, Propylenglykol, Glyzerin und ähnlichen derartigen Verbindungen bzw. Kombinationen Verwendung finden.

Für den Fall der parenteralen Anwendung können Lösungen des Wirkstoffes in Sesam- oder Erdnußöl oder in wäßrigem Propylenglykol oder N,N-Dimethylformamid eingesetzt werden. Als sehr wirksam und praktisch hat sich die topische (pour on, spot on) Verabreichung von Lösungen bei Haustieren erwiesen.

Die neuen Verbindungen können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die Wirkstoffe können in neuartiger Weise angewendet werden, insbesondere sind sie für die Applikation mit dem Trinkwasser oder Futter bestimmt.

Die besonderen Eigenschaften erlauben es, die Behandlung bei Geflügel an nur wenigen (4 bis 8) Tagen anstatt der sonst üblichen Dauermedikation über 6 - 8 Wochen anzuwenden.

Als Dosierungen für die Praxis kommen bei der Behandlung von Geflügel, vor allem Hühner, Enten, Gänse und Truthühner, Zumischungen von 5 - 100 ppm, vorzugsweise

10 - 100 ppm, zum Futter oder Lösungen/Suspensionen in Trinkwasser in Frage, die in speziellen Fällen aufgrund der guten Verträglichkeit erhöht werden können.

Für die Einzelbehandlung z.B. bei der Säugetierbehandlung hat es sich als vorteilhaft erwiesen, Mengen von etwa 5 bis etwa 250 mg/kg Körpergewicht pro Tag zur Erzielung wirksamer Ergebnisse zu verabreichen. Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art der Formulierung und dem Zeitpunkt bzw. Intervall, zu dem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten auch die weiteren obigen Ausführungen.

Folgende Versuche mögen die hier genannten Eigenschaften und völlig neuen Behandlungswege beispielhaft belegen:

Beispiel A

Intervallbehandlung beim Geflügel

Eintagsküken (Mast-Typ /englisch = broiler/) wurden in folgenden Gruppen für 51 Tage in Bodenhaltung gehalten:

1. unbehandelt, nicht experimentell infiziert (20 Tiere)
2. unbehandelt, experimentell infiziert (30 Tiere)
3. nicht experimentell infiziert (30 Tiere) Behandlung mit Trinkwasser, das 25 ppm 1-/3-Methyl-4-(4'-trifluormethylthio-phenoxy)-phenyl/-3-methyl-1,3,5-triazin-2,4,6-(1H,3H,5H)-trion enthält
4. experimentell infiziert (20 Tiere) Behandlung mit Trinkwasser, das 25 ppm 1-/3-Methyl-4-(4'-trifluormethylthio-phenoxy)-phenyl/-3-methyl-1,3,5-triazin-2,4,6-(1H,3H,5H)-trion enthält
5. Monensin = Coccidiosemittel zu Vergleichszwecken eingesetzt experimentell infiziert (20 Tiere) Behandlung mit Futter, das 125 ppm Monensin enthielt vom ersten bis zum letzten Versuchstag
6. Monensin = Coccidiosemittel zu Vergleichszwecken eingesetzt nicht experimentell infiziert (20 Tiere) Behandlung mit Futter, das 125 ppm Monesin enthielt vom ersten bis zum letzten Versuchstag.

Die Tiere der Gruppen 2, 4 und 5 wurden experimentell mittels Schlundsonde an den Lebenstagen 6, 20 und 36 mit den oben genannten Inokula infiziert. Die Infektionsdosen je Tier betrugen bei jeder Infektion in etwa für

| | | |
|---|---|---|
| Eimeria acervulina | 12.700 sporulierte | Oocysten |
| Eimeria maxima | 10.700 " | " |
| Eimeria tenella | 20.800 " | " |

Die Tiere waren untergebracht in Boxen von ca. 1,25 x 1,25 m Bodenfläche. Die Boxen wurden während der Versuchsdauer nicht gereinigt. Lediglich in den Boxen der nicht experimentell infizierten Kontrollgruppen wurde 1 x wöchentlich die Einstreu gewechselt. Die Raumtemperatur betrug je nach Alter der Tiere im allgemeinen 26-28°C. Während der ersten zwei Lebenswochen wurden zusätzlich Wärmestrahler aufgehängt. Futter und Wasser standen den Tieren ad libitum zur Verfügung.

Die Tiere der Gruppen 3 und 4 wurden an den Tagen 8, 9, 15, 16, 22, 23 behandelt.

Die Tiere der Gruppen 5 und 6 wurden vom ersten bis letzten Versuchstag über das Futter behandelt. Als Kontrollgruppen dienten jeweils eine infizierte, unbehandelte Kontrolle und eine nicht experimentell infizierte Kontrolle.

Auswertung:

Berücksichtigt wurden:

- Gewichtsentwicklung vom 1. bis letzten Versuchstag durch Wägung in wöchentlichem Abstand (Tabelle 1).

- Oocystenausscheidung: an den Tagen 11-13, 25-27 und 42-44 wurden Gitterroste (ca. 40 x 60 x 4 cm) in die Boxen gelegt, nachdem die alte Einstreu an dieser Stelle zuvor zur Seite geschoben worden war. Am folgenden Tag wurde in dem darunter gefundenen Kot mittels McMasterkammer die Anzahl ausgeschiedener Oocysten je Gramm Kot (OpG) bestimmt (Tabelle 2).

Tabelle 1 Gewichtsveränderung während des Versuchs

| Gruppe | durchschnittliches Körpergewicht sowie Standardabweichungen davon am gegebenen Lebenstag | | | | | |
|---|---|---|---|---|---|---|
| | 13 | 20 | 27 | 35 | 41 | 51 Tag |
| 1 | 151<br>8 | 294<br>13 | 464<br>26 | 763<br>131 | 1047<br>178 | 1492<br>140 |
| 2 | 127<br>9 | 215<br>21 | 439<br>25 | 749<br>113 | 890<br>138 | 1449<br>164 |
| 3 | 145<br>11 | 273<br>39 | 489<br>28 | 799<br>83 | 1080<br>112 | 1607<br>92 |
| 4 | 152<br>8 | 287<br>18 | 499<br>30 | 830<br>105 | 1133<br>123 | 1545<br>150 |
| 5 | 147<br>10 | 276<br>16 | 499<br>36 | 809<br>112 | 1108<br>198 | 1537<br>159 |
| 6 | 141<br>15 | 259<br>32 | 459<br>50 | 718<br>95 | 981<br>129 | 1395<br>148 |

Tabelle 2: Oocystenausscheidung

| Gruppe | Oocystenanzahl pro Gramm Kot am angegebenen Tag | | | | Eimeria |
|---|---|---|---|---|---|
| | 11-13 | 25-27 | 42-44 | | |
| 1 | 0<br>0<br>0 | 218.000<br>98.000<br>30.000 | 1.800<br>200<br>0 | | a**<br>m<br>t |
| 2 | 196.000<br>140.000<br>20.000 | 22.200<br>13.400<br>4.600 | 0<br>0<br>0 | | a<br>m<br>t |
| 3 | 0<br>0<br>0 | 0<br>0<br>0 | 1.800<br>1.600<br>200 | | a<br>m<br>t |
| 4 | 0<br>0<br>0 | 0<br>0<br>0 | 2.800<br>800<br>1.000 | | a<br>m<br>t |
| 5 | 4.800<br>4.400<br>1.200 | 0<br>0<br>0 | 200<br>400<br>200 | | a<br>m<br>t |
| 6 | 0<br>0<br>0 | 0<br>0<br>0 | 0<br>0<br>0 | | a<br>m<br>t |

** a = aceryulina
m = maxima
t = tenella

Beispiel B

Substanzen, die die körpereigene Abwehr (Immunsystem) während einer Infektion stimulieren, sind sowohl für die Human- wie für die Veterinärmedizin von großem Interesse, da viele Infektionen trotz guter chemotherapeutischer Möglichkeiten ohne Unterstützung durch körpereigene Abwehrmechanismen persistieren. Dies kann zum erneuten Auftreten von Symptomen (Rezidiv) nach dem Überstehen der Ersterkrankung und damit zu chronisch rezidivierenden Krankheiten führen. Unter den durch Bakterien bedingten Erkrankungen sind es besonders Infektionen mit sogenannten fakultativ intrazellulären Bakterien, die Probleme darstellen. Darunter versteht man Bakterien, die sich nach Aufnahme in die Makrophagen (Phagozytose) weiter vermehren. Es bedarf erst einer weiteren Stimulierung der Makrophagen über das Immunsystem, um diese intrazellulären Bakterien abzutöten. Ein Experimentalmodell für eine solche Erkrankung stellt die Infektion der Maus mit Salmonella typhimurium dar. Nach Inokulation der Mäuse mit diesem humanpathogenen Bakterium kommt es in Abhängigkeit von der Infektionsdosis zu einem subakut bis chronischen Krankheitsverlauf, bei dem die Tiere erst nach 4 bis 7 Tagen abzusterben beginnen. Während dieses Zeitraumes besteht die Möglichkeit, das Immunsystem durch Substanzen zu beeinflussen. Bei den meisten anderen tierexperimentellen Infektionen, kommt es zu einem sehr schnellen Absterben der Tiere innerhalb von 1 bis 2 Tagen. Damit besteht keine Möglichkeit mehr, die Abwehr während der Infektion zu stimulieren.

 0158075

1-[3-Methyl-4-(4'-trifluormethylthio-phenoxy)-phenyl]-3-methyl-1,3,5-triazin-trion wurde Mäusen an 4 aufeinanderfolgenden Tagen einmal täglich per os in unterschiedlichen Dosen verabfolgt, beginnend 1 Tag nach intraperitonealer Infektion mit 7 x $10^5$ Kolonien-bildenden Einheiten von Salmonella typhimurium. Diese Infektionsdosis führt bei unbehandelten Tieren innerhalb von 4 bis 6 Tagen zu einem Absterben von 70 bis 90 % der Tiere. Die Tiere wurden in Macrolon-Käfigen unter konstanten Bedingungen (22° ± 2°C; 55-65 % relative Luftfeuchtigkeit) gehalten und erhielten Sniff Versuchstierdiät.

In mehreren Versuchen (Beispiel s. Tabelle) kam es nach Behandlung der Tiere mit 10 mg/kg Wirkstoff zu einer Verzögerung des Beginns der Absterbephase (6.Tag noch 90 % überlebende Mäuse) und zu einer Erhöhung der Überlebensrate. Am 18. Tag lebten noch 60 % der Mäuse. Da die Substanz in vitro keinen direkten Effekt auf die Vermehrung von Salmonella typhimurium zeigte, ist anzunehmen, daß Abwehrmechanismen des Wirtes gesteigert werden. Dies führt zu einer Verzögerung des Beginns der Absterbephase und zu einer Erhöhung der Überlebensrate.

Tabelle 3

Überlebensrate von Mäusen zu verschiedenen Zeiten nach intraperitonealer Infektion mit Salmonella typhimurium unter Behandlung mit 1-[3-Methyl-4-(4'-trifluormethylthio-phenoxy)-phenyl]-3-methyl-1,2,5-triazin-trion im Vergleich zu unbehandelten Kontrollen

| Dosis mg/kg Kg | Überlebende Mäuse (%) an folgenden Tagen nach Infektion 6. Tag | 12. Tag | 18. Tag |
|---|---|---|---|
| 10 | 90 | 60 | 60 |
| Kontrollen | 45 | 30 | 25 |

## Patentansprüche

1. Immunstimulierende Mittel, gekennzeichnet durch einen Gehalt an Verbindungen der Formel I

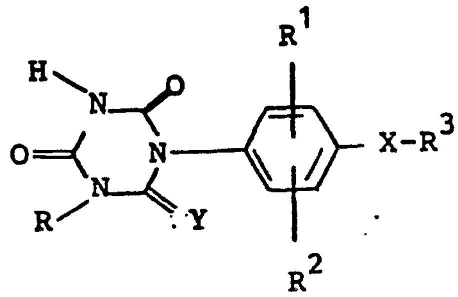

(I)

in welcher

R für Alkyl steht,

$R^1$ für Wasserstoff, Alkyl, Halogen, Halogenalkyl, Alkoxy steht,

$R^2$ für Wasserstoff, Alkyl, Halogen, Halogenalkyl, Alkoxy steht,

$R^3$ für gegebenenfalls substituiertes Aryl oder Heteroaryl steht,

X für O oder S steht,

Y für O oder S steht.

2. Immunstimulierende Mittel gemäß Anspruch 1 gegen human- und tierpathogene Viren, Bakterien, Protozoen oder Tumoren bei Mensch und Tier.

3. Immunstimulierende Mittel gemäß Anspruch 1 gegen Coccidien.

4. Immunstimulierende Mittel gemäß Anspruch 1 gegen tierpathogene Coccidien bei Geflügel und Säugetieren.

5. Verwendung der Mittel gemäß Ansprüche 1 bis 4 zur Stimulierung der körpereigenen Abwehr.

6. Verwendung der Mittel gemäß Ansprüche 1 bis 4 bei Geflügel und Säugetieren in Form einer Intervallbehandlung nur an nur ein bis zwei aufeinanderfolgenden Tagen pro Woche in den für die Infektion besonders empfänglichen Lebensphasen.

7. Verwendung der Mittel gemäß Ansprüche 1 bis 4 bei Geflügel in Form einer Intervallbehandlung an ein bis zwei aufeinanderfolgenden Tagen in der zweiten, dritten, vierten Lebenswoche.

8. Verwendung der Mittel gemäß Ansprüche 1 bis 4 bei Säugetieren in Form einer Intervallbehandlung an einem Tag pro Woche ab der vierten bis sechsten Lebenswoche.

9. Verfahren zur Stimulierung der körpereigenen Abwehr, dadurch gekennzeichnet, daß man Mittel gemäß Ansprüche 1 bis 4 appliziert.

Europäisches Patentamt

# EUROPÄISCHER RECHERCHENBERICHT

0158075
Nummer der Anmeldung

EP 85 10 2045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | US-A-4 219 552 (BAYER AG) * Ansprüche * | 3,4 | A 61 K 31/53<br>C 07 D 251/34<br>C 07 D 251/38<br>C 07 D 417/12 |
| D,X | DE-A-2 413 722 (BAYER AG) * Ansprüche * | 3,4 | |
| X | DE-A-2 650 014 (BAYER AG) * Ansprüche * | 3,4 | |
| | | | RECHERCHIERTE SACHGEBIETE (Int. Cl.4) |
| | | | A 61 K 31/00<br>C 07 D 251/00 |

Der vorliegende Recherchenbericht wurde für alle Patentansprüche erstellt

| Recherchenort | Abschlußdatum der Recherche | Prüfer |
|---|---|---|
| DEN HAAG | 13-06-1985 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument